(19) **Europäisches Patentamt**
**European Patent Office**
**Office européen des brevets**

(11) **EP 3 835 370 A1**

(12) **EUROPÄISCHE PATENTANMELDUNG**

(43) Veröffentlichungstag:
**16.06.2021 Patentblatt 2021/24**

(51) Int Cl.:
*C09C 1/00* (2006.01)      *C08K 9/04* (2006.01)
*A61K 8/19* (2006.01)      *C08K 9/02* (2006.01)
*C09D 11/00* (2014.01)      *D21H 17/67* (2006.01)

(21) Anmeldenummer: 20213109.0

(22) Anmeldetag: **10.12.2020**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
**KH MA MD TN**

(30) Priorität: **11.12.2019 DE 102019008593**

(71) Anmelder: **Merck Patent GmbH**
**64293 Darmstadt (DE)**

(72) Erfinder:
• **Schmitt, Adeliene**
**67056 LUDWIGSHAFEN (DE)**
• **Kaviratna, Padma**
**PHILADELPHIA, PA 19112, Georgia (US)**
• **Gumsheimer, Udo**
**64293 DARMSTADT (DE)**
• **Tellefsen, Mark**
**GIBBSTOWN, NJ 08027, Georgia (US)**
• **Cheng, Qingmin**
**PHILADELPHIA, PA 19112, Georgia (US)**

(54) **MIT KOHLENSTOFF BESCHICHTETE BIOCL-PIGMENTE**

(57) Die vorliegende Erfindung betrifft mit amorphem Kohlenstoff beschichtete BiOCl-Pigmente, ein Verfahren zur Herstellung der Pigmente sowie die Verwendung der so hergestellten Pigmente u.a. in Farben, Lacken, Druckfarben, Kunststoffen und in der Kosmetik.

**EP 3 835 370 A1**

**Beschreibung**

Die vorliegende Erfindung betrifft mit amorphem Kohlenstoff beschichtete plättchenförmige BiOCl-Pigmente, ein Verfahren zur Herstellung der Pigmente sowie die Verwendung der so hergestellten Pigmente u.a. in Farben, Lacken, Druckfarben, Kunststoffen und in der Kosmetik.

Metalleffektpigmente werden seit vielen Jahren in Beschichtungen zur Erzeugung von metallischen Effekten eingesetzt, beispielsweise in Druckfarben oder in Metallic-Lackierungen für Automobile. Klassische Metalleffektpigmente bestehen aus plättchenförmigen Metallpartikeln, deren optische Wirkung auf der gerichteten Reflexion von einfallendem Licht an der in idealer Weise flächig und plan ausgebildeten Oberfläche der Metallpartikel beruht, die im jeweiligen Anwendungsmedium parallel zur Oberfläche ausgerichtet vorliegen.

Die weltweit am häufigsten eingesetzten Typen von Metalleffektpigmenten bestehen aus Aluminium, daneben sind noch Kupfer- und Kupfer/ZinkPigmente oder auch Zinkpigmente im Einsatz. Form, Dicke, Größe und Oberflächengüte der Metallpigmente werden durch das Herstellverfahren bestimmt.

Dünne Metalleffektpigmente, insbesondere Aluminiumplättchen, werden eingesetzt, wenn man in Lackierungen, wie z.B. Automobillacken, einen sogenannten "Liquid Metal"-Effekt realisieren möchte. Aluminium-Pigment-Plättchen sind herstellungsbedingt sehr teuer und müssen auf Grund ihrer intrinsisch reaktiven Oberfläche mit hohem sicherheitstechnischem Aufwand in allen Applikationen gehandhabt werden. Die AluminiumPigment-Plättchen werden in Lacksysteme dispergiert und anschließend auf die zu lackierende Oberfläche mittels Sprühapplikation oder elektrostatischer Applikation aufgetragen. Zur Erreichung des "Liquid Metal"-Effekts ist in der Regel eine 7-Schicht-Applikation notwendig. Nach dem Aufbringen einer jeden Aluminium-Pigmentplättchen enthaltenden Lackschicht, muss die aufgebrachte Schicht erst trocknen, damit eine Orientierung der Pigmente planparallel zu beschichteten Oberfläche erreicht wird. Auf Grund dieser hohen Rohstoff- und Handhabungskosten für das Pigment und der komplexen Speziallackierung (7-Schicht-Aufbau inklusive separater Trocknungsschritte) beschränkt sich die "Liquid Metal"-Effekt-Lackierung für den Automobil-Bereich auf teure Premium-Produkte/ Premium-Automobile.

Daher werden für Sonderlackierungen im Automobil-Bereich oft günstigere und sicherheitstechnisch ungefährliche Interferenz-/ Farbwechsel-Pigmente eingesetzt. Allerdings sind Effektpigmente, wie z.B. Interferenz-Pigmente, Farbwechsel-Pigmente, basierend auf anorganischen Substraten um ein Vielfaches dicker als die intrinsisch dünnen Metall-Pigmentplättchen und eignen sich daher in der Regel nicht für eine "Liquid Metal"-Effekt-Lackierung. Erschwerend kommt hinzu, dass die Pigment-PartikelOberfläche von Interferenz-Pigmenten infolge des Herstellungsprozesses Rauigkeiten auf der Oberfläche aufweist, die als Streuzentren für diffuses Licht fungieren. Das eintreffende Licht kann also an der Oberfläche nicht, wie im Vergleich zu einer ideal glatten (Spiegel)-Oberfläche, reflektiert werden. Folglich zeigt eine Lackierung enthaltend Interferenz-Pigmente keinen ausgeprägten "Liquid Metall"-Effekt.

Eine Lackierung zeigt den sogenannten "Liquid Metal"-Effekt, wenn das äußere Erscheinungsbild einem silberfarbenem Metall in einer flüssigen Schmelze oder einem Metallüberzug aus einem Metallisierungsbad gleicht. Die Lackierung wird vom Betrachter als texturlos, absolut homogen und metallisch-silbrig glänzend wahrgenommen. Ferner wird die Oberfläche aus jeder Betrachtungsrichtung als äußerst brillant empfunden, ohne Glanz- oder Helligkeitsdifferenzen, wie z.B. einer Matt-/Glanzänderung oder einem Hell-/Dunkel-Flop infolge eines sich ändernden Betrachtungswinkels. Nur durch die planparallele Orientierung der Pigmente in jeder Lackschicht erscheint die finale "Liquid Metal"-Lackierung als homogen und texturlos und erinnert an eine "flüssige" Metall-Oberfläche.

Die Forderung nach der Texturlosigkeit bedingt, dass die Pigmente, die im Lacksystem dispergiert werden, plättchenförmig sind und eine Dicke aufweisen, die nicht größer ist als die Dicke der Lackschicht. Idealerweise sollten die Pigmente eine Dicke von < 60 nm aufweisen.

Ein Effektpigment mit einer sehr hohen Brillanz ist plättchenförmiges Bismutoxychlorid (BiOCl). Der metallisch silberweiße Glanz und die physiologische Unbedenklichkeit haben BiOCl in der dekorativen Kosmetik unentbehrlich gemacht. Die geringe Lichtstabilität - kurzwelliges Licht färbt BiOCl grau -, das schnelle Absetzverhalten sowie eine geringe mechanische Belastbarkeit sind von Nachteil von BiOCl-Pigmenten in industriellen Einsatzgebieten, wie z. B. Industrielacke, Automobillacke, Farben, Kunststoffe, etc. Zur Erhöhung des Deckvermögens wird BiOCl-Pigmenten häufig ein schwarzes Additiv zugemischt, wie z.B. Carbon Black oder schwarzes Eisenoxid. Die Zumischung der Additive erhöht zwar das Deckvermögen, führt aber zu weiteren Nachteilen, wie z.B. gelbstichiges Erscheinungsbild, Entmischungsphänomene, Stabilitätsprobleme einer physikalischen Mischung, Magnetismus beim Einsatz von Eisenoxiden.

Es besteht daher der Bedarf den hohen metallischen Glanz von plättchenförmigen BiOCl-Pigmenten für alle Anwendungen zu nutzen und die oben genannten Nachteile zu beheben. Aufgabe der vorliegenden Erfindung ist, es Licht-stabile und hoch-reflektive plättchenförmige BiOCl-Effektpigmente mit metallischer Optik zur Verfügung zu stellen, die neben dem hohen Glanz auch ein hohes Deckvermögen aufweisen, und sich in allen Anwendungsmedien wie z. B. Lacken, Druckfarben, Kunststoffen und in der Kosmetik leicht verarbeiten lassen. Das BiOCl-Pigment soll weiterhin in Lackierungen die Generierung eines ausgeprägten "Liquid Metal"-Effektes in einfacher Weise ermöglichen.

Überraschenderweise wurde gefunden, dass BiOCl-Pigmente, die mit einer dünnen amorphen Schicht aus Kohlenstoff beschichtet sind, ein metallisches Erscheinungsbild mit hohem Glanz zeigen und gleichzeitig die UV-Stabilität der Pigmente deutlich erhöht wird. Weiterhin zeigen die metallisch glänzenden Pigmente ein sehr hohes Deckvermögen.

Gegenstand der Erfindung sind beschichtete BiOCl-Pigmente, dadurch gekennzeichnet, dass plättchenförmige BiOCl-Pigmente auf der Oberfläche mit einer amorphen Kohlenstoffschicht beschichtet sind.

Die erfindungsgemäßen stabilen Pigmente sind nicht elektrisch leitfähig, weisen eine attraktive silberne Interferenzfarbe sowie einen sehr hohen Glanz auf und besitzen gegenüber den unbeschichteten BiOCl-Pigmenten ein deutlich höheres Deckvermögen.

Gegenstand der Erfindung ist auch ein Verfahren zur Herstellung der erfindungsgemäßen BiOCl-Pigmente, sowie deren Verwendung u.a. in Lacken, Farben, Kunststoffen und in der Kosmetik. Gegenstand der Erfindung ist auch ein einfaches Lack-Applikationsverfahren unter Einsatz der erfindungsgemäßen Pigmente zur Herstellung von Lackierungen, die einen attraktiven "Liquid Metal"-Effekt zeigen.

Als Substrat für das erfindungsgemäße Pigment kommen alle dem Fachmann bekannten plättchenförmigen BiOCl-Pigmente als Pulver in Betracht. Häufig werden BiOCl-Pigmente nur in Form von Pasten im Handel angeboten, z. B. als pastöse Form in Castor Oil oder Rizinusöl. Die Pasten enthaltend BiOCl-Pigmente sind als Substrat für die Kohlenstoff-beschichtung ungeeignet.

Die BiOCl-Plättchen haben vorzugsweise Partikelgrößen von 1 - 30 $\mu$m, insbesondere 1 - 25 $\mu$m und ganz besonders bevorzugt von 5 - 15 $\mu$m.

Die Dicke der BiOCl-Plättchen beträgt vorzugsweise 50 - 100 nm, insbesondere 60 - 80 nm und ganz besonders bevorzugt 40 - 60 nm.

Als Teilchengröße oder Partikelgröße wird die größte Ausdehnung der Pigmentplättchen in Länge und Breite bezeichnet.

Dabei liegt der volumengewichtete $D_{50}$-Wert vorzugsweise bei $\leq$ 25 $\mu$m, insbesondere 5-20 $\mu$m und ganz besonders bevorzugt 5-10 $\mu$m. Der volumengewichtete $D_{90}$-Wert liegt vorzugsweise bei $\leq$ 50 $\mu$m, insbesondere 30-50 $\mu$m und ganz besonders bevorzugt 35-45 $\mu$m.

Die volumengewichteten $D_{90}$- bzw. $D_{50}$-Werte geben jeweils an, dass 90 beziehungsweise 50 Volumenprozent der Pigmente einer Pigmentschüttung eine Teilchengröße aufweisen, die unterhalb des genannten Wertes liegt.

Die Wahl der Teilchengröße der erfindungsgemäßen BiOCl-Pigmente hat einen Einfluss auf die finalen Eigenschaften, da eine geringe Teilchengröße, verbunden mit einem sehr hohen Feinanteil, ausschlaggebend ist für ein erhöhtes Deckvermögen der Pigmente in den jeweiligen Anwendungsmedien, während die einzelnen Pigmentpartikel als solche semitransparent sind, also lediglich ein mittleres Deckvermögen in der Anwendung erwarten lassen.

Die Teilchengröße und die Teilchengrößenverteilung können über verschiedene fachübliche Methoden ermittelt werden. Bevorzugt wird erfindungsgemäß jedoch die Laserbeugungsmethode in einem Standardverfahren mittels eines Malvern Mastersizer MS 3000 der Fa. Malvern Instruments Ltd., UK eingesetzt. Diese Verfahren hat den Vorteil, dass Partikelgröße und Partikelgrößenverteilung gleichzeitig unter Standardbedingungen bestimmt werden können.

Die Partikelgröße sowie die Dicke von Einzelpartikeln lässt sich außerdem mit Hilfe von SEM (Scanning Electron Microscope) Bildern ermitteln. Bei diesen können Partikelgröße und geometrische Partikeldicke über direktes Ausmessen ermittelt werden. Zur Ermittlung von Durchschnittswerten werden mindestens 1000 Partikel einzeln ausgewertet und die Ergebnisse gemittelt.

Die erfindungsgemäßen BiOCl-Pigmente weisen vorzugsweise einen Formfaktor (Verhältnis von Länge bzw. Breite zu Dicke) von 10-600, vorzugsweise 60-400, auf.

Geeignete BiOCl-Plättchen in Form von Pulver sind kommerziell erhältlich z. B. von der Fa. Merck KGaA unter den Markennamen RonaFlair® LF-2000, RonaFlair® B-50, RonaFlair® ESQ, RonaFlair® Fines, RonaFlair® MTU, und von der Fa. BASF SE unter dem Markennamen Mearlite® MBU.

Auf die plättchenförmigen BiOCl-Pigmente (= Substrate) wird eine dünne amorphe Kohlenstoffschicht aufgebracht. Diese amorphe Kohlenstoffschicht ist nicht elektrisch leitfähig und umhüllt vollständig das Substrat. Bei der Kohlenstoffschicht handelt es sich um eine kompakte geschlossene Schicht auf dem Substrat. Die geometrische Dicke der Kohlenstoffschicht ist vorzugsweise 1 - 5 nm, insbesondere 1 - 3 nm und ganz besonders bevorzugt 1 - 2 nm. Diese nur wenige Nanometer dicke Kohlenstoffschicht erhöht deutlich das Deckvermögen der transparenten BiOCl-Pigmente, ohne aber den Glanz und die Reflektivität der Substrate negativ zu beeinflussen.

Der Anteil an amorphen Kohlenstoff bezogen auf das beschichtete BiOCl-Pigmente beträgt vorzugsweise 1 - 5 Gew.%, insbesondere 1 - 3 Gew.% und ganz besonders bevorzugt 1 - 2 Gew.% bezogen auf die Masse des beschichteten Pigments.

Beim Aufbringen von konformalen, kompakten, amorphen und homogenen Kohlenstoff-Schichten auf die partikulären BiOCl-Substrate muss jedes einzelne BiOCl-Plättchen mit einer dünnen Kohlenstoff-Schicht umhüllt werden, um ungewollte Streuzentren zu vermeiden. Die Kohlenstoff-Beschichtung partikulärer Systeme wird üblicherweise durch eine aufwändige nasschemische Hydrothermal-Synthese in Anwesenheit von hohen Drücken und Temperaturen aufgebracht oder durch in der thermalen Gasphase stattfindenden Chemical Vapour Deposition (CVD-Verfahren) realisiert. Die dünnen BiOCl-Plättchen sind allerdings nur bis zu einer Temperatur von maximal 350-400 °C thermisch stabil, bevor die Zersetzung des BiOCl und die Bildung von $Bi_2O_3$ einsetzt.

Die Beschichtung der plättchenförmigen BiOCI-Pigmente mit der Kohlenstoffschicht findet vorzugsweise in einem Reaktor statt. Als Reaktoren, in denen das erfindungsgemäße Verfahren durchgeführt werden kann, sind sowohl Drehrohröfen als auch Wirbelbettreaktoren geeignet, wobei letztere bevorzugt eingesetzt werden. Das Beschichtungsverfahren wird mit Hilfe eines Trägergasstrom durchgeführt. Während der Beschichtung werden die BiOCI-Pigmente in Bewegung gehalten.

Als Trägergas werden Inertgase eingesetzt. Beispielhaft für Inertgase können Stickstoff und Argon genannt werden, wobei Stickstoff bevorzugt eingesetzt wird.

Der Kohlenstoff für die Kohlenstoffbeschichtung kann aus pulverförmigen oder leicht verdampfbaren, also gasförmigen Kohlenstoffquellen (=C-Quellen) stammen.

Bei pulverförmigen C-Quellen, wird wie folgt verfahren:
Das BiOCI-Pigment wird extern mit Hilfe eines geeigneten Mischers oder im Reaktor mit ein oder mehreren pulverförmingen Kohlenstoff-haltigen Verbindungen gemischt. Diese sind vorzugsweise ausgewählt aus der Gruppe der Mono-, Di- und Trisaccharide, vorzugsweise Fructose, Glucose (=Dextrose), Galaktose, Xylose, Mannose, Lactose, Saccharose, Maltose oder deren Gemische. In einer bevorzugten Ausführungsform wird Saccharose in Form von Puderzucker eingesetzt.

Bei gasförmigen C-Quellen, wie z.B. Aceton oder Ethin (Acetylen), wird die C-Quelle vorzugsweise dem Trägergas beigemischt.

Das Trägergas kann jedoch auch selbst aus der gasförmigen Kohlenstoff-haltigen Verbindung bestehen, die in diesem Falle sowohl die Funktion des Trägergases als auch die der gasförmigen Kohlenstoff-haltigen Verbindung übernimmt.

Der Einsatz von pulverförmigen C-Quellen ist bevorzugt. In einer ganz besonders bevorzugten Ausführungsform ist die C-Quelle Puderzucker.

Die Aufbringung der einzelnen dünnen amorphen Kohlenstoffschichten auf die BiOCI-Plättchen findet daher vorzugsweise in einem Wirbelschicht basierenden CVD-Verfahren unter Einsatz von reaktiven/ thermisch instabilen Kohlenstoff-Präkursoren und bei milden Reaktionstemperaturen, vorzugsweise bei 150 - 400 °C, insbesondere bei 200 - 350 °C und ganz besonders bevorzugt bei 180 - 250 °C statt. Wirbelschicht-Reaktoren haben sich auf Grund ihrer exzellenten Wärme- und Stofftransporteigenschaften sowie ihrer guten Skalierbarkeit bewährt. Die vergleichsweise niedrigen Reaktionstemperaturen führen bei der thermischen Zersetzung des Kohlenstoff-Präkursors bzw. der Kohlenstoffpräkursoren zu einer Abscheidung von amorphen Kohlenstoffschichten auf den BiOCI-Plättchen.

In einer bevorzugten Ausführungsform werden die trockenen BiOCI-Plättchen physikalisch in einem externen Mischer, wie z. B. einem Turbula-, Rhönrad-Mischer, mit feinen partikulär vorliegenden Monosacchariden, wie z. B. handelsüblicher Glukose (Dextrose), Fructose oder Galaktose, wie auch mit Disacchariden, z.B. Maltose oder Saccharose (Puderzucker), etc., im Rahmen eines sogenannten "Dry-Particle"-Coating gemischt. Über vander-Waals-Kräfte bleiben die feinen Zucker-Partikel, wie z. B. Saccharid-Partikel, (= Guest-Partikel) an der BiOCI-Pigment-Oberfläche (= Host-Partikel) haften.

Das Herstellen der Mischung von Sacchariden und BiOCI-Pigment-Plättchen in einem definierten Verhältnis muss dabei nicht zwingend wie oben beschrieben in einem externen Mischer stattfinden, sondern kann auch in einer Wirbelschicht realisiert werden.

Ferner ist das Aufbringen eines wässrigen Kohlenstoffpräkursors, wie z.B. einer wässrigen Saccharidlösung, im Sprühtrockner oder über einen Verdampfer direkt auf die Oberfläche des BiOCI-Plättchens möglich.

Die Einsatzmenge des Kohlenstoff-Präkursors bezogen auf das Substrat beträgt vorzugsweise 1-5 Gew.%, insbesondere 2-5 Gew.%, und ganz besonders bevorzugt 1-3 Gew.%. Ein Überangebot an Kohlenstoff-Präkursor kann zu unerwünschten, festen Nebenprodukten führen, die das finale Erscheinungsbild der beschichteten BiOCI-Pigmente beeinträchtigen können.

In einer bevorzugten Ausführungsform werden mit Saccharid-Partikel konditionierte BiOCI-Plättchen vorzugsweise im Wirbelbettverfahren zur Reaktion gebracht.

Zunächst werden sie unter Inertgas-Atmosphäre in einem Wirbelschichtreaktor fluidisiert. Die Durchmischung der Partikel wird durch das inerte Fluidisationsgas erreicht, und kann durch den Einsatz von Fluidisationsunterstützungen, wie Vibrationen, etc., verbessert werden. Auf diese Weise wird zudem die Agglomeration der Partikeln und Kanalbildung innerhalb der Wirbelschicht verringert. Abhängig von der angestrebten Kohlenstoff-Schichtdicke wird das verwendete Saccharid in entsprechender Menge thermisch zersetzt. Die notwendige Wärme wird über die Wirbelschicht-Reaktorwand in die fluidisierte BiOCI/Saccharid-Schüttung eingetragen. Nach einer bestimmten Reaktionszeit von 5 bis 30 min und bei Reaktionstemperaturen von 180 - 250 °C ist die CVD-Reaktion bzw. die Pyrolyse des Saccharids soweit fortgeschritten, dass die zu beschichtenden BiOCI-Pigment-Plättchen konformal und "pinhole"-frei mit einer dünnen amorphen Kohlenstoff-Schicht beschichtet vorliegen.

Unter ständiger Inertgas-Zufuhr werden die beschichteten BiOCI-Pigment-Plättchen auf Raumtemperatur gekühlt und schließlich aus der Wirbelschicht entnommen.

Die Kohlenstoff-Schichtdicke wird durch die Kohlenstoff-Präkursormenge, z. B. die zugegebene Saccharidmenge, determiniert.

Die Morphologie des abgeschiedenen Kohlenstoffs ist amorph, was mit Hilfe der konfokalen Raman-Spektroskopie bestätigt wird.

Gegenstand der vorliegenden Erfindung ist auch die Verwendung der erfindungsgemäßen Pigmente in Farben, Lacken, Druckfarben, Beschichtungszusammensetzungen, Kunststoffen, Folien, in der Kosmetik, Knopfpasten, in Trockenpräparaten und Pigmentpräparationen.

Bedingt durch ihr metallisches Erscheinungsbild mit hohem Glanz und silberner Interferenzfarbe sowie das außerordentlich hohe Deckvermögen sind die erfindungsgemäßen Pigmente sehr gut geeignet zur Pigmentierung von Anwendungsmedien. Dabei werden sie in der gleichen Weise wie übliche Interferenzpigmente eingesetzt.

Die Feinteiligkeit der erfindungsgemäßen BiOCl-Pigmente macht sehr geringe Schichtdicken des jeweiligen Anwendungsmediums, beispielsweise im Bereich von 2-20 $\mu$m, möglich, ohne dass beim Deckvermögen der Beschichtung qualitative Abstriche gemacht werden müssen. Daher sind die erfindungsgemäßen Pigmente insbesondere für den Einsatz in Industrie- und Automobillacken geeignet.

In Lacken werden die erfindungsgemäßen Pigmente vorzugsweise in Mengen von 20-50 Gew.% bezogen auf den Lack eingesetzt und in Effektlackierungen in Mengen von 8-30 Gew.% bezogen auf den Effektlack. Die eingesetzte Konzentration ist dabei abhängig vom gewünschten Farbton.

Die erfindungsgemäßen BiOCl-Pigmente zeigen in Lackierungen einen ausgeprägten "Liquid Metal"-Effekt.

Es hat sich insbesondere gezeigt, dass der "Liquid Metal"-Effekt besonders gut ausgeprägt ist, wenn statt des bei Metalleffektpigmenten bekannten 7-stufigen Lackierverfahrens ein sequentielles dreistufiges Lackieren mit jeweils nachgeschalteten Trocknungsschritten eingesetzt wird.

Die mit Kohlenstoff beschichteten BiOCl-Plättchen werden entweder in einem Wasser- oder Lösungsmittel-basierendem "Basecoat" dispergiert. Anschließend wird die Suspension auf der zu lackierenden Oberfläche sequentiell appliziert. Nach jedem Auftrag wird der soeben aufgetragene "Basecoat" für 1 - 15 Minuten, vorzugsweise 5 Minuten, bei einer Temperatur von 60 - 90 °C, vorzugsweise 80 °C getrocknet. Die Trocknungszeiten hängen von dem verwendeten Lacksystem ab. Erst nach dem beschriebenen Trocknungsschritt erfolgt die Applikation der zweiten "Basecoat"-Schicht. Insgesamt wird dieser Schritt dreimal wiederholt. Abgeschlossen wird die Lackierung mit einer Klarlack-Schicht, in die z. B. ein oder mehrere Additive, wie z.B. UV-Stabilisator(en), eingebracht werden können.

LIDAR / RADAR-Sensoren werden bereits jetzt zur Fahrerunterstützung eingesetzt und sind für das autonome Fahren essentiell. Aluminium-basierende Pigmente in der Autolackierung haben den Nachteil, dass Aluminium-basierende Pigmente für RADAR-Signale nicht durchlässig sind. Als Alternative können z. B. die mit Kohlenstoff beschichteten BiOCl-Pigmente verwendet werden, da diese sich ebenfalls durch eine hohe Reflektivität im sichtbaren Bereich auszeichnen und in Folge der Kohlenstoff-Beschichtung ein verbessertes Deckvermögen aufweisen, und zudem RADAR durchlässig sind.

Beim Einsatz der beschichteten BiOCl-Pigmente in Lacken und Farben sind alle dem Fachmann bekannten Anwendungsbereiche möglich, wie z.B. Pulverlacke, Automobillacke, Druckfarben für den Tief-, Offset-, Sieb-, oder Flexodruck sowie Lacke in Außenanwendungen. Für die Herstellung von Druckfarben ist eine Vielzahl von Bindern, insbesondere wasserlösliche, UV-härtende, aber auch lösemittelhaltige Typen, z.B. auf der Basis von Acrylaten, Methacrylaten, Polyestern, Polyurethanen, Nitrocellulose, Ethylcellulose, Polyamid, Polyvinylbutyrat, Phenolharzen, Melaminharzen, Maleinharzen, Stärke oder Polyvinylalkohol geeignet. Bei den Lacken kann es sich um Wasser- oder lösemittelbasierte und UV-härtende Lacke handeln, wobei die Auswahl der Lackbestandteile dem Allgemeinwissen des Fachmannes unterliegt.

Die erfindungsgemäßen BiOCl-Pigmente können ebenso vorteilhaft zur Herstellung von Kunststoffen und Folien eingesetzt werden, und zwar für alle dem Fachmann bekannten Anwendungen. Als Kunststoffe eignen sich dabei alle gängigen Kunststoffe, beispielsweise Duromere, Elastomere und thermoplastische Kunststoffe.

Die erfindungsgemäßen BiOCl-Pigmente sind auch zur Herstellung von fließfähigen Pigmentpräparationen und Trockenpräparaten geeignet, die ein oder mehrere erfindungsgemäße Pigmente, gegebenenfalls weitere Pigmente bzw. Farbmittel, Bindemittel und optional ein oder mehrere Additive enthalten. Unter Trockenpräparaten sind auch Präparate zu verstehen, die 0 bis 8 Gew.-%, vorzugsweise 2 bis 8 Gew.-%, insbesondere 3 bis 6 Gew.-%, an Wasser und/oder eines Lösemittels oder Lösemittelgemisches enthalten. Die Trockenpräparate liegen vorzugsweise als Pearlets, Pellets, Granulate, Chips, oder Briketts vor und weisen Teilchengrößen von etwa 0,2 bis 80 mm auf.

Die Konzentration der erfindungsgemäßen BiOCl-Pigmente im jeweiligen Anwendungsmedium ist von den dort gewünschten Eigenschaften bezüglich Farbgebung und Glanz abhängig und kann vom Fachmann jeweils auf der Basis üblicher Rezepturen gewählt werden. Vorzugsweise werden die erfindungsgemäßen Pigmente im Kunststoff in Mengen von 0,5 - 5 Gew.-% (Spritzguss: 0,5 - 1 Gew.%, dünne Folien 8 Gew.%) bezogen auf den Kunststoff, in der Druckfarbe in Konzentrationen von 10 - 25 Gew.-% bezogen auf die Druckfarbe, in der Kosmetik in Konzentrationen von 0,01-100 Gew.-%, vorzugsweise 2 - 30 Gew.-% bezogen auf die kosmetische Formulierung, eingesetzt.

Obwohl die erfindungsgemäßen BiOCl-Pigmente über attraktive optische Eigenschaften verfügen und damit als alleinige Effektpigmente in verschiedensten Anwendungen einsetzbar sind, ist es selbstverständlich möglich und je nach Anwendungszweck auch vorteilhaft, sie bei Bedarf mit organischen und/oder anorganischen Farbmitteln (insbesondere mit Weiß- oder Buntpigmenten) zu mischen oder gemeinsam mit diesen in einer Anwendung, beispielsweise einer Beschichtung, einzusetzen.

Die Mischungsverhältnisse bei allen vorab beschriebenen Mischungen sind nicht limitiert, solange die vorteilhaften Eigenschaften der erfindungsgemäßen Pigmente durch die zugemischten Fremdpigmente nicht negativ beeinflusst werden. Die erfindungsgemäßen Pigmente können in jedem Verhältnis mit anwendungsüblichen Zusatzstoffen, Füllstoffen und/oder Bindemittelsystemen gemischt werden.

Als plättchenförmige Farbmittel kommen vor allem Perlglanzpigmente insbesondere auf Basis von natürlichem oder synthetischem Glimmer, $SiO_2$-Plättchen oder $Al_2O_3$-Plättchen, die nur mit einer Metalloxidschicht umhüllt sind, Metalleffektpigmente (auf beispielsweise Al-Plättchen, Fe-Plättchen, Bronzen), optisch variable Pigmente (OVP's), Flüssigkristallpolymerpigmente (LCP's) oder holographische Pigmente in Frage.

Zu den sphärischen Farbmitteln zählen insbesondere $TiO_2$, eingefärbtes $SiO_2$, $CaSO_4$, Eisenoxide, Chromoxide, Ruß, Pflanzenruß, organische Farbpigmente, wie z.B. Anthrachinon-Pigmente, Chinacridon-Pigmente, Diketopyrrolo-pyrrol-Pigmente, Phthalocyanin-Pigmente, Azopigmente, Isoindolin-Pigmente. Bei den nadelförmigen Pigmenten handelt es sich vorzugsweise um eingefärbte Glasfasern, $\alpha$-FeOOH, organische Farbpigmente, wie z.B. Azopigmente, $\beta$-Phthalocyanin Cl Blue 15,3, Cromophtalgelb 8GN (Ciba-Geigy), Irgalith Blau PD56 (Ciba-Geigy), Azomethinkupferkomplex Cl Yellow 129, Irgazingelb 5GT (Ciba-Geigy).

Die erfindungsgemäßen BiOCl-Pigmente können weiterhin mit handelsüblichen Füllstoffen gemischt werden. Als Füllstoffe sind z.B. zu nennen natürlicher und synthetischer Glimmer, Natriumkaliumaluminiumsilikat, Glasbeads oder Glaspulver, Nylon Powder, reine oder gefüllte Melaminharze, Talkum, Gläser, Kaolin, Oxide oder Hydroxide von Aluminium, Magnesium, Calcium, Zink, BiOCl, Bariumsulfat, Calciumsulfat, Calciumcarbonat, Magnesiumcarbonat, Kohlenstoff, sowie physikalische oder chemische Kombinationen dieser Stoffe.

Bezüglich der Partikelform des Füllstoffes gibt es keine Einschränkungen. Sie kann den Anforderungen gemäß z. B. plättchenförmig, sphärisch, nadelförmig, kristallin oder amorph sein.

Die Einsatzkonzentration und das Mischungsverhältnis der erfindungsgemäßen BiOCl-Pigmente insbesondere mit organischen und anorganischen Farbpigmenten und Farbstoffen, natürlichen oder synthetischen Ursprungs, wie z.B. Chromoxid, Ultramarin, sphärischen $SiO_2$- oder $TiO_2$-Pigmenten, sind abhängig vom Anwendungsmedium und dem Effekt, der erzielt werden soll.

Die erfindungsgemäßen BiOCl-Pigmente können sowohl vorteilhaft in der dekorativen als auch in der pflegenden Kosmetik eingesetzt werden, wie z.B. in Lippenstiften, Lipgloss, Eyeliner, Lidschatten, Rouge, Sonnenschutz, Prä-Sun- und After-Sun-Präparate, Make-ups, Body Lotions, Badegele, Seifen, Badesalze, Zahnpasta, Haargele, (Volumen)Mascara, Nagellacke, Presspuder, Shampoos, lose Puder und Gele, etc.

Die Konzentration der erfindungsgemäßen BiOCl-Pigmente im zu pigmentierenden Anwendungssystem liegt in der Regel zwischen 0,01 und 70 Gew.%, vorzugsweise zwischen 0,1 und 50 Gew.% und insbesondere zwischen 1,0 und 10 Gew.%, bezogen auf den Gesamtfestkörpergehalt des Systems. Sie ist in der Regel abhängig vom konkreten Anwendungsfall und kann bei losen Pudern bis zu 100 % betragen. Die Einsatzkonzentration des erfindungsgemäßen BiOCl-Pigments reicht von 0,01 Gew.% im Shampoo bis zu 70 Gew. % im Presspuder. Bei einer Mischung der erfindungsgemäßen BiOCl-Pigmente mit sphärischen Füllstoffen, z. B. $SiO_2$, $TiO_2$, kann die Konzentration bei 0,01-70 Gew.% in der Formulierung liegen. Die kosmetischen Produkte, wie z.B. Nagellacke, Lippenstifte, Presspuder, Shampoos, lose Puder und Gele, zeichnen sich durch ihren metallischen Glanz und ihr verbessertes Deckvermögen aus.

Selbstverständlich können die erfindungsgemäßen BiOCl-Pigmente in den Formulierungen auch mit jeder Art von kosmetischen Roh- und Hilfsstoffen kombiniert werden. Dazu gehören u.a. Öle, Fette, Wachse, Filmbildner, Tenside, Antioxidantien, wie z.B. Vitamin C oder Vitamin E, Stabilisatoren, Geruchsverstärker, Silikonöle, Emulgatoren, Lösemittel wie z.B. Ethanol, oder Ethylacetat oder Butylacetat, Konservierungsmittel und allgemein anwendungstechnische Eigenschaften bestimmende Hilfsstoffe, wie z.B. Verdicker und rheologische Zusatzstoffe wie etwa Bentonite, Hektorite, Siliciumdioxide, Ca-Silicate, Gelatinen, hochmolekulare Kohlenhydrate und/oder oberflächenaktive Hilfsmittel, etc.

Ebenso können nanoskalige Dielektrika zur Verbesserung des Hautgefühls beigemischt werden. Beispiele für derartige Beimischungen sind $Al_2O_3$, $SiO_2$, ZnO oder $TiO_2$, die üblicherweise in Mengen von 0,01 - 15 % der Formulierung zugegeben werden.

Die die erfindungsgemäßen BiOCl-Pigmente enthaltenden Formulierungen können dem lipophilen, hydrophilen oder hydrophoben Typ angehören. Bei heterogenen Formulierungen mit diskreten wässrigen und nicht-wässrigen Phasen können die erfindungsgemäßen Pigmentgemische in jeweils nur einer der beiden Phasen enthalten oder auch über beide Phasen verteilt sein.

Die pH-Werte der Formulierungen können zwischen 1 und 14, bevorzugt zwischen 2 und 11 und besonders bevorzugt zwischen 5 und 8 liegen.

Den Konzentrationen der erfindungsgemäßen BiOCl-Pigmente in der Formulierung sind keine Grenzen gesetzt.

Sie können - je nach Anwendungsfall - zwischen 0,001 (rinse-off-Produkte, z.B. Duschgele) - 100 % (z.B. Glanzeffekt-Artikel für besondere Anwendungen) liegen.

Die erfindungsgemäßen BiOCl-Pigmente können weiterhin auch mit kosmetischen Wirkstoffen kombiniert werden. Geeignete Wirkstoffe sind z.B. Insect Repellents, anorganische UV-Filter, wie z.B. $TiO_2$, UV A/BC-Schutzfilter (z.B. OMC, B3, MBC), auch in verkapselter Form, Anti-Aging-Wirkstoffe, Vitamine und deren Derivate (z.B. Vitamin A, C, E, etc.), Selbstbräuner (z.B. DHA, Erythrulose, u.a.) sowie weitere kosmetische Wirkstoffe, wie z.B. Bisabolol, LPO, VTA, Ectoin, Emblica, Allantoin, Bioflavanoide und deren Derivate.

Organische UV-Filter werden in der Regel in einer Menge von 0,5 bis 10 Gewichtsprozent, vorzugsweise 1 bis 8 %, anorganische Filter von 0,1 bis 30% in kosmetische Formulierungen eingearbeitet.

Die kosmetischen Zubereitungen enthaltend das erfindungsgemäße BiOCl-Pigment können darüber hinaus weitere übliche hautschonende oder hautpflegende Wirkstoffe enthalten. Dies können prinzipiell alle den Fachmann bekannten Wirkstoffe sein. Besonders bevorzugte Wirkstoffe sind Pyrimidincarbonsäuren und/oder Aryloxime.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Up, Pflegecremes und Sonnenschutzpräparaten eingesetzt werden.

Als Anwendungsform der kosmetischen Formulierungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isethionate, Lanolin, Fettalkohole, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie z.B. Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Die kosmetischen Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-In-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsionen werden besonders bevorzugt. Emulsionen, W/O-Emulsionen und O/W-Emulsionen sind in üblicher Weise erhältlich.

Weitere Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glykols, wie Propylenglykol, und/oder eines Polyols, wie Glycerin, und Ölen, Wachsen und Fettsäureestern, wie Triglyceriden von Fettsäuren, dar.

Feste Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen photochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shamponieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung mit Lichtschutzeigenschaften kann Adjuvantien enthalten, wie Grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Formulierungen enthaltend das erfindungsgemäße BiOCl-Pigment können weiterhin mindestens einen Bestandteil ausgewählt aus der Gruppe Absorptionsmittel, Adstringenzien, antimikrobielle Stoffe, Antioxidantien, Antiperspirantien, Antischaummittel, Antischuppenwirkstoffe, Antistatika, Bindemittel, biologische Zusatzstoffe, Bleichmittel, Chelatbildner, Desodorierungsmittel, Emollentien, Emulgatoren, Emulsionsstabilisatoren, Farbstoffe, Feuchthaltemittel, Filmbildner, Füllstoffe, Geruchsstoffe, Geschmacksstoffe, Insect Repellents, Konservierungsmittel, Korrosionsschutzmittel, kosmetischen Öle, Lösungsmittel, Oxidationsmittel, pflanzliche Bestandteile, Puffersubstanzen, Reduktionsmittel, Tenside, Treibgase, Trübungsmittel, UV-Filter, UV-Absorber, Vergällungsmittel, Aloe Vera, Avocadoöl, Coenzym Q10, grüner Tee Extrakt, Viskositätsregler, Parfüme, Vitamine, Enzyme, Spurenelemente, Proteine, Kohlenhydrate, organische Pigmente, anorganische Pigmente, einschließlich Effektpigmente, enthalten.

Die vorliegende Erfindung wird durch die nachfolgenden Beispiele, auf die sie jedoch nicht beschränkt sein soll, näher erläutert.

**Beispiele**

Beispiel 1

In einem Turbula-Mischer (Fa. WAB) werden 100 g getrocknete plättchenförmige BiOCl-Pigmente der Teilchengröße < 20 $\mu$m und einer Dicke von 60 - 80 nm, $D_{50}$ = 15 $\mu$m und $D_{80}$ = 20 $\mu$m (Malvern Mastersizer MS 3000) mit 2 g Saccharose (Puderzucker) für 30 min bei einer Frequenz von 34 U/min gemischt. Die Kohlenstoff-Beschichtung wird in einem Wirbelschicht-Reaktor (DI = 100 mm) bei einer Reaktionstemperatur von 250 °C durchgeführt. Als Fluidisationsgas wird $N_2$ verwendet. Die Fluidisationsgeschwindigkeit entspricht dem 10-fachen Wert der theoretisch zu erwartenden Minimalfluidisationsgeschwindigkeit von 2 mm/s. Die Kohlenstoff-Beschichtung findet unter inerten Bedingungen für 30 min statt. Danach wird die fluidisierte BiOCl-Partikel-Schüttung ebenfalls unter Inertgas auf Raumtemperatur abgekühlt. Im Anschluss an die Kohlenstoff-Beschichtung erfolgt eine Siebung.

Das erhaltene mit amorphen Kohlenstoff beschichtete BiOCl-Pigment zeichnet sich durch einen metallisch silbrig-grauen Glanz aus.

Beispiel 2

In einem Turbula-Mischer (Fa. WAB) werden 100 g getrocknete plättchenförmige BiOCl-Pigmente der Teilchengröße < 20 $\mu$m und einer Dicke von 50 - 80 nm, $D_{50}$ = 15 $\mu$m und $D_{80}$ = 20 $\mu$m (Malvern Mastersizer MS 3000) mit 1 g Saccharose (Puderzucker) für 30 min bei einer Frequenz von 72 U/min. gemischt. Die Kohlenstoff-Beschichtung wird in einem Wirbelschicht-Reaktor (DI = 100 mm) bei einer Reaktionstemperatur von 200 °C durchgeführt. Als Fluidisationsgas wird $N_2$ verwendet. Die Fluidisationsgeschwindigkeit entspricht dem 10-fachen Wert der theoretisch zu erwartenden Minimalfluidisationsgeschwindigkeit von 2 mm/s. Die Kohlenstoff-Beschichtung findet unter inerten Bedingungen für 30 min statt. Danach wird die fluidisierte BiOCl-Partikel-Schüttung ebenfalls unter Inertgas auf Raumtemperatur abgekühlt. Im Anschluss an die Kohlenstoff-Beschichtung erfolgt eine Siebung.

Das Pigment gemäß Beispiel 2 zeigt einem im Vergleich zu Beispiel 1 dunkleren metallischen silbrig-grauen Glanz.

Beispiel 3

In einem Turbula-Mischer (Fa. WAB) werden 100 g plättchenförmige BiOCl-Pigmente vom Typ RonaFlair® LF-2000 (Fa. Merck, BiOCl-Pigment mit $D_{50}$ = 8 - 20 $\mu$m, $D_{80}$ < 35 $\mu$m, Dicke 200 - 500 nm) mit 3 g D-Fructose für 30 min bei einer Frequenz von 200 U/min. gemischt. Die Kohlenstoff-Beschichtung wird in einem Wirbelschicht-Reaktor (DI = 100 mm) bei einer Reaktionstemperatur von 220 °C durchgeführt. Als Fluidisationsgas wird $N_2$ verwendet. Die Fluidisationsgeschwindigkeit entspricht dem 10-fachen Wert der theoretisch zu erwartenden Minimalfluidisationsgeschwindigkeit von 2 mm/s. Die Kohlenstoff-Beschichtung findet unter inerten Bedingungen für 20 min statt. Danach wird die fluidisierte BiOCl-Partikel-Schüttung ebenfalls unter Inertgas auf Raumtemperatur abgekühlt. Im Anschluss an die Kohlenstoff-Beschichtung erfolgt eine Siebung.

Man erhält ein dunkles silberweiß glänzendes Pigment.

Beispiel 4

In einem Turbula-Mischer (Fa. WAB) werden 100 g plättchenförmige BiOCl-Pigmente vom Typ RonaFlair® B-50 (2 - 35 $\mu$m, $D_{50}$ = 9 - 15 $\mu$m, $D_{80}$ = 2 - 35 $\mu$m, Dicke 200 - 500 nm) mit 2,5 g Glucose für 30 min bei einer Frequenz von 150 U/min. gemischt. Die Kohlenstoff-Beschichtung wird in einem Wirbelschicht-Reaktor (DI = 100 mm) bei einer Reaktionstemperatur von 150 °C durchgeführt. Als Fluidisationsgas wird $N_2$ verwendet. Die Fluidisationsgeschwindigkeit entspricht dem 10-fachen Wert der theoretisch zu erwartenden Minimalfluidisationsgeschwindigkeit von 2 mm/s. Die Kohlenstoff-Beschichtung findet unter inerten Bedingungen für 30 min statt. Danach wird die fluidisierte BiOCl-Partikel-Schüttung ebenfalls unter Inertgas auf Raumtemperatur abgekühlt. Im Anschluss an die Kohlenstoff-Beschichtung erfolgt eine Siebung.

Man erhält ein dunkles silberweiß glänzendes Pigment.

Beispiel 5

In einem Turbula-Mischer (Fa. WAB) werden 100 g plättchenförmige BiOCl-Pigmente vom Typ RonaFlair® ESQ (Produkt der Fa. Merck KGaA mit der Teilchengröße 2 - 35 $\mu$m, $D_{50}$ = 11 -17 $\mu$m, $D_{80}$ = 2 - 35 $\mu$m, Dicke 200 - 500 nm) mit 5 g Lactose für 40 min bei einer Frequenz von 45 U/min. gemischt.

Die Kohlenstoff-Beschichtung wird in einem Wirbelschicht-Reaktor (DI = 100 mm) bei einer Reaktionstemperatur von 280 °C durchgeführt. Als Fluidisationsgas wird $N_2$ verwendet. Die Fluidisationsgeschwindigkeit entspricht dem 10-fachen Wert der theoretisch zu erwartenden Minimalfluidisationsgeschwindigkeit von 2 mm/s. Die Kohlenstoff-Beschichtung findet unter inerten Bedingungen für 30 min statt. Danach wird die fluidisierte BiOCl-Partikel-Schüttung ebenfalls unter Inertgas auf Raumtemperatur abgekühlt. Im Anschluss an die Kohlenstoff-Beschichtung erfolgt eine Siebung.

Man erhält ein sehr dunkles silberglänzendes Pigment.

Beispiel 6

In einem Turbula-Mischer (Fa. WAB) werden 100 g plättchenförmige BiOCl-Pigmente vom Typ RonaFlair® Fines (Produkt der Fa. Merck mit der Teilchengröße 2 - 35 $\mu$m, $D_{50}$ = 9 - 15 $\mu$m, $D_{80}$ = 2 - 35 $\mu$m, Dicke 50 - 100 nm) mit 1 g Glucose für 30 min bei einer Frequenz von 60 U/min. gemischt. Die Kohlenstoff-Beschichtung wird in einem Wirbelschicht-Reaktor (DI = 100 mm) bei einer Reaktionstemperatur von 250 °C durchgeführt. Als Fluidisationsgas wird $N_2$ verwendet. Die Fluidisationsgeschwindigkeit entspricht dem 10-fachen Wert der theoretisch zu erwartenden Minimalfluidisationsgeschwindigkeit von 2 mm/s. Die Kohlenstoff-Beschichtung findet unter inerten Bedingungen für 30 min statt. Danach wird die fluidisierte BiOCl-Partikel-Schüttung ebenfalls unter Inertgas auf Raumtemperatur abgekühlt. Im Anschluss an die Kohlenstoff-Beschichtung erfolgt eine Siebung.

Man erhält ein helles silberglänzendes Pigment.

Beispiel 7

Es werden 100 g plättchenförmige BiOCl-Pigmente vom Typ RonaFlair® MTU (Produkt der Firma Merck KGaA mit der Teilchengröße 2 - 35 $\mu$m, Dicke 500 - 700 nm) mit 2,5 g Saccharose (Puderzucker) in einem Wirbelschicht-Reaktor (DI = 100 mm) für 30 min bei Minimalfluidisationsgeschwindigkeit gemischt. Die Kohlenstoff-Beschichtung wird in dem Wirbelschicht-Reaktor bei einer Reaktionstemperatur von 180 °C durchgeführt. Als Fluidisationsgas wird $N_2$ verwendet. Die Fluidisationsgeschwindigkeit entspricht dem 10-fachen Wert der theoretisch zu erwartenden Minimalfluidisationsgeschwindigkeit von 2 mm/s. Die Kohlenstoff-Beschichtung findet unter inerten Bedingungen für 30 min statt. Danach wird die fluidisierte BiOCl-Partikel-Schüttung ebenfalls unter Inertgas auf Raumtemperatur abgekühlt. Im Anschluss an die Kohlenstoff-Beschichtung erfolgt eine Siebung.

Man erhält ein helles silberglänzendes Pigment.

Die "pinhole"-freie, amorphe Kohlenstoff-Beschichtung der BiOCl-Plättchen wird mit der konfokalen RAMAN-Spektroskopie sowie mit Hilfe von Transmissionselektronen-Mikrokopie (TEM) nachgewiesen.

Die mit amorphem Kohlenstoff beschichteten BiOCl-Pigmente zeichnen durch eine erhöhte Stabilität, durch einen metallisch silbernen Glanz und eine hohe Deckkraft aus und lassen sich sehr gut in Anwendungsmedien dispergieren und redispergieren.

**Anwendungsbeispiele**

Anwendungsbeispiel 1: Lack

20,35 g BiOCl-Pigment aus Beispiel 1 wird in 679,65 g eines kommerziell erhältlichen wasserbasierten Basislacks (Mipa WBC000, Fa. Mipa) eingearbeitet. Die Trocknung erfolgt bei 80 °C für 5 min. Der pigmentierte Lack wird auf ein Blech appliziert. Anschließend wird mit 805 g 2K-Klarlacks (Mipa CC4, Fa. Mipa) überlackiert. Die Trocknung findet für 15 min bei Raumtemperatur statt und anschließend bei 80 °C für 5 min.

Anwendungsbeispiel 2: Lack

20,35 g BiOCl-Pigment aus Beispiel 2 wird in 679,65 g eines kommerziell erhältlichen wasserbasierten Basislacks (Mipa WBC000, Fa. Mipa) eingearbeitet. Die Trocknung erfolgt bei 80 °C für 5 min. Der pigmentierte Lack wird auf ein Blech appliziert. Anschließend wird mit 805 g 2K-Klarlacks (Mipa CC4, Fa. Mipa) überlackiert.

Die Trocknung findet für 15 min bei Raumtemperatur statt und anschließend bei 80 °C für 5 min.

Der "Liquid Metal"-Effekt der lackierten Bleche wird gemäß Gleichung 1 unter Einsatz eines BYK-mac i der Firma BYK Gardner GmbH untersucht. Es handelt sich um ein Mehrwinkel-Farbmessgerät.

Messtechnisch erfolgt die Bewertung des "Liquid Metal"-Effekts einer Lackierung durch den Liquid-Index LI, der als das Verhältnis von Flop Index FI und Körnigkeit/ Graininess G definiert ist und gemäß:

$$LI = \frac{FI}{G} = \frac{\frac{(L_{15°}^{*} - L_{110°}^{*})^{1,11}}{L_{45}^{*\ 0,86}}}{G} \qquad (I)$$

berechnet wird.

FI beschreibt den Helligkeitsunterschied L* im flachen (L*(15 °)) und steilen Betrachtungswinkel (L*(110°)). Die Lacktextur wird durch die Größe G (Körnigkeit/ Graininess) beschrieben. Ein schichtartiger Lackauftrag, unabhängig von der Anwendung im Automobil- oder kosmetischen Lack, zeigt den "Liquid Metal"-Effekt nur für den Fall, dass FI größer als 21 ist. Gleichzeitig muss G Werte kleiner drei aufweisen, so dass der LI für den Fall einer vorliegenden "Liquid Metal"-Lackierung stets größer als sieben ist (siehe Messwerte für Beispiel 1 in Tabelle).

|  | **FI** (Flop Index) | **G** (Graininess) | **LI** (Liquid Index) |
|---|---|---|---|
| beschichtetes BiOCl-Pigment aus Beispiel 1 | 21,7 | 2,4 | 8,9 |
| unbeschichtetes BiOCl-Pigment aus Beispiel 1 | 18,5 | 2,8 | 6,6 |

Die erfindungsgemäßen Pigmente gemäß der Beispiele 1 und 2 zeigen im Lack einen ausgeprägten "Liquid Metal"-Effekt.

Anwendungsbeispiel 3 - Eye Shadow

| Rohstoff<br>Phase A | | INCI (CTFA) | Gew.-% |
|---|---|---|---|
| Pigment gemäß Beispiel 1 | (1) | | 30,00 |
| Parteck® LUB Talc | (1) | TALC | 10,00 |
| Phase B | | | |
| RonaCare® AP | (1) | BIS-ETHYLHEXYL HYDROXYDIMETHOXY BENZYLMALONATE | 0,50 |

(fortgesetzt)

| Phase B | | | |
|---|---|---|---|
| Oxynex® K liquid | (1) | PEG-8, TOCOPHEROL, ASCORBYL PALMITATE, ASCORBIC ACID, CITRIC ACID | 0,10 |
| all-rac-alpha-Tocopheryl acetate | (1) | TOCOPHERYL ACETATE | 0,50 |
| Parteck® LUB STA 50 | (1) | STEARIC ACID | 3,00 |
| SP Crodamol PMP MBAL-LQ-(MH) | (2) | PPG-2 MYRISTYL ETHER PROPIONATE | 30,90 |
| Syncrowax HGLC | (2) | C18-36 ACID TRIGLYCERIDE | 10,00 |
| Miglyol® 812 N | (3) | CAPRYLIC/CAPRIC TRIGLYCERIDE | 8,00 |
| Syncrowax HRC | (2) | TRIBEHENIN | 3,00 |
| Ganex™ V-216 | (4) | PVP/HEXADECENE COPOLYMER | 2,00 |
| Sunflower Oil, refined | (5) | HELIANTHUS ANNUUS SEED OIL (HELIANTHUS ANNUUS (SUNFLOWER) SEED OIL) | 1,00 |
| Sensiva® PA 20 | (6) | PHENETHYL ALCOHOL, ETHYLHEXYL GLYCERIN | 1,00 |

Herstellung:

Phase B auf 80 °C erhitzen bis alle Bestandteile geschmolzen sind. Abkühen auf 65 °C und unter Rühren Zugabe der Bestandteile von Phase A. Abfüllen der Masse in das gewünschte Behältnis bei 65 °C. Abkühlen auf Raumtemperatur.

Hersteller:

(2) Croda
(3) IOI Oleo GmbH          (4) Ashland
(5) Gustav Heess GmbH   (6) Schülke & Mayr GmbH

Anwendungsbeispiel 4 - Eye Shadow Gel

Phase A

| Rohstoff | Bezugsquelle | INCI | Gew.-% |
|---|---|---|---|
| Pigment gemäß Beispiel 2 | Merck KGaA/Rona® | | 15,00 |
| Micronasphere® M | Merck KGaA/Rona® | MICA, SILICA | 8,00 |
| Carbopol Ultrez 21 | Noveon | ACRYLATES/C10-30 ALKYL ACRYLATE CROSSPOLYMER | 0,40 |
| Citronensäure Monohydrat | Merck KGaA/Rona® | CITRIC ACID | 0,00 |
| Wasser | | AQUA (WATER) | ad 100 |

Phase B

| Rohstoff | Bezugsquelle | INCI | Gew.-% |
|---|---|---|---|
| Glycerin | Merck KGaA/Rona® | GLYCERIN | 3,00 |
| Konservierungsmittel | | | q.s. |
| Triethanolamin | | TRIETHANOLAMINE | 0,70 |
| Wasser | | AQUA (WATER) | 13,00 |

Phase C

| Rohstoff | Bezugsquelle | INCI | Gew.-% |
|---|---|---|---|
| Lubrajel DV | | PROPYLENE GLYCOL, POLYGLYCERYL-METHACRYLATE | 5,00 |

Herstellung:

Pigment und Micronasphere® im Wasser der Phase A dispergieren. Mit einigen Tropfen Citronensäure ansäuern um die Viskosität zu vermindern, Carbopol unter Rühren einstreuen. Nach vollständiger Lösung die vorgelöste Phase B langsam einrühren und anschließend Phase C. Zum Schluss den pH-Wert zwischen 7,0 - 7, 5 einstellen.

Anwendungsbeispiel 5 - Lippenstift

Phase A

| Rohstoff | Bezugsquelle | INCI | Gew.-% |
|---|---|---|---|
| Pigment gemäß Beispiel 2 | | | 12,00 |
| Ronastar® Purple Sparks | Merck KGaA/Rona® | Calcium Aluminum Borosilicate, CI77891 (Titanium dioxide), Silica, Tin Oxide | 3,00 |

Phase B

| Rohstoff | Bezugsquelle | INCI | Gew.-% |
|---|---|---|---|
| Bienenwachs | Merck KGaA/Rona® | Cera Alba (Beeswax) | 8,75 |
| Paracera C44 | Paramelt | COPERNICIA CERIFERA (CARNAUBA WAX), CERESIN | 5,25 |
| Adeps Lanae | Henry Lamotte GmbH | LANOLIN | 3,50 |
| Isopropylmyristat | Cognis GmbH | Isopropyl Myristate | 5,60 |
| Paraffin dickflüssig | Merck KGaA/Rona® | PARAFFINUM LIQUIDUM (MINERAL OIL) | 2,10 |
| Rizinusöl | Henry Lamotte GmbH | RICINUS COMMUNIS (CASTOR OIL) | 59,65 |
| Oxynex® K flüssig | Merck KGaA/Rona® | PEG-8, TOCOPHEROL, ASCORBYL PALMITATE ASCORBIC ACID, CITRIC ACID | 0,05 |
| Propyl-4-hydroxybenzoat | Merck KGaA/Rona® | PROPYLPARABEN | 0,10 |

Herstellung:

Die Bestandteile der Phase B werden auf 75 °C erhitzt und aufgeschmolzen. Die Pigmente der Phase A werden zugegeben und alles gut durchrührt. Die Lippenstiftmasse wird dann in der auf 65 °C temperierten Gießapparatur 15 Minuten gerührt. Die homogene Schmelze wird in die auf 55 °C vorgewärmten Gießform gegossen. Anschließend kühlt man die Formen ab und entfernt die Gießlinge kalt. Nach Erwärmen der Lippenstifte auf Raumtemperatur werden die Lippenstifte kurz abgeflammt.

Anwendungsbeispiel 6 - Nagellack

| Rohstoff | Bezugsquelle | INCI | Gew.-% |
|---|---|---|---|
| Pigment gemäß Beispiel 3 | | | 2,00 |
| Nailsyn® Sterling 60 Silver | Merck KGaA/ Rona® | CI 77163 (Bismuth Oxychloride), Butyl Acetate, Nitrocellulose, Isopropyl Alcohol, Ethyl Acetate, Stearnalkonium Hectorite | 1,00 |
| Thixotrope Nagellack -Base 155 | Durlin/ Bergerac NC | BUTYL ACETATE, ETHYL ACETATE NITROCELLULOSE, ACETYL TRIBUTYL CITRATE, PHTHALIC ANHYDRIDE/TRIME LLITIC ANHYDRIDE/GLYCO LS COPOLYMER, ISOPROPYL ALCOHOL, STEARALKONIUM HECTORITE, ADIPIC ACID/FUMARIC ACID/ PHTHALIC ACID/TRICYCLODE CANE DIMETHANOL COPOLYMER, CITRIC ACID | 97,00 |

Herstellung:

Das Pigment und Nailsyn® Sterling 60 Silver werden zusammen mit der Lackbase eingewogen, gut mit einem Spatel von Hand vermischt und anschließend 10 min bei 1000 Upm gerührt.

Anwendungsbeispiel 7 - Seife

| Rohstoff | Bezugsquelle | INCI | Gew.-% |
|---|---|---|---|
| Pigment gemäß Beispiel 1 | | | 1,50 |
| Ronastar® Noble Sparks | Merck KGaA/ Rona® | Calcium Aluminum Borosilicate, Silica, CI 77891 (Titanium Dioxide), Tin Oxide | 0,50 |
| Transparente Seifen base | Jean Charles (USA) | SODIUM PALMATE, SODIUM LAURETH SULFATE, SODIUM STEARATE, SODIUM MYRISTATE, SODIUM COCOYL ISETHIONATE, TRIETHANOLAMINE , AQUA (WATER), GLYCERIN, SORBITOL, PROPYLENE GLYCOL, FRAGRANCE | 98,00 |

Herstellung:

Alle Bestandteile werden homogen gemischt.

**Patentansprüche**

1. Beschichtete BiOCl-Pigmente, **dadurch gekennzeichnet, dass** plättchenförmige BiOCl-Pigmente auf der Oberfläche mit einer amorphen Kohlenstoffschicht beschichtet sind.

2. Beschichtete BiOCl-Pigmente nach Anspruch 1, **dadurch gekennzeichnet, dass** die plättchenförmigen BiOCl-Pigmente eine Partikelgröße von 1 - 30 $\mu$m aufweisen.

3. Beschichtete BiOCl-Pigmente nach Anspruch 1 oder 2, **dadurch gekennzeichnet, dass** die BiOCl-Pigmente eine Dicke von 50 - 100 nm aufweisen.

4. Beschichtete BiOCl-Pigmente nach einem oder mehreren der Ansprüche 1 bis 3, **dadurch gekennzeichnet, dass** die BiOCl-Pigmente einen volumengewichteten $D_{50}$-Wert von < 25 $\mu$m (bestimmt mit Malvern) aufweisen.

5. Beschichtete BiOCl-Pigmente nach einem oder mehreren der Ansprüche 1 bis 4, **dadurch gekennzeichnet, dass** die BiOCl-Pigmente einen Formfaktor (Verhältnis von Länge bzw. Breite zu Dicke) von 10 - 600 aufweisen.

6. Beschichtete BiOCl-Pigmente nach einem oder mehreren der Ansprüche 1 bis 5, **dadurch gekennzeichnet, dass** die amorphe Kohlenstoffschicht eine geometrische Schichtdicke von 1 bis 5 nm aufweist.

7. Beschichtete BiOCl-Pigmente gemäß einem oder mehreren der Ansprüche 1 bis 6 **dadurch gekennzeichnet, dass** der Anteil der amorphen Kohlenstoffschicht bezogen auf das Gewicht des beschichteten BiOCl-Pigments 0,5 - 5 Gew.-% beträgt.

8. Verfahren zur Herstellung der beschichteten BiOCl-Pigmente nach einem oder mehreren der Ansprüche 1 bis 7, **dadurch gekennzeichnet, dass** plättchenförmige BiOCl-Pigmente als Substrat in einem Reaktor in einem Trägergasstrom bei Temperaturen von 150-400 °C in Gegenwart einer oder mehrerer Kohlenstoff-haltiger Verbindungen durch pyrolytische Zersetzung der Kohlenstoff-haltigen Verbindung(en) mit einer Schicht aus amorphen Kohlenstoff belegt werden.

9. Verfahren nach Anspruch 8, **dadurch gekennzeichnet, dass** die Kohlenstoff-haltige Verbindung Aceton, Ethin oder eine pulverförmige Verbindung ausgewählt aus der Gruppe der Mono-, Di- oder Trisaccharide ist.

10. Verfahren nach Anspruch 8 oder 9, **dadurch gekennzeichnet, dass** die Kohlenstoff-haltige Verbindung Fructose, Glucose (=Dextrose), Galaktose, Xylose, Mannose, Lactose, Saccharose, Maltose oder ein Gemisch der genannten Verbindungen ist.

11. Verfahren nach einem oder mehreren der Ansprüche 8 bis 10, **dadurch gekennzeichnet, dass** die plättchenför-migen BiOCl-Pigmente in dem Reaktor in Bewegung gehalten werden.

12. Verwendung der beschichteten BiOCl-Pigmente nach einem oder mehreren der Ansprüche 1 bis 7 in Farben, Lacken, Industrie- und Automobillacken, Druckfarben, Papier, Kunststoffen, Folien, kosmetischen Formulierungen, Knopfpasten, RADAR-durchlässigen Beschichtungen, Trockenpräparaten oder Pigmentpräparationen.

13. Verwendung der beschichteten BiOCl-Pigmente nach oder mehreren der Ansprüche 1 bis 7 in einem Dreischicht-Lackiersystem.

14. Formulierungen enthaltend das beschichtete BiOCl-Pigment nach einem oder mehreren der Ansprüche 1 bis 7.

15. Formulierungen nach Anspruch 14, **dadurch gekennzeichnet, dass** es sich bei den Formulierungen um Industrielacke und Automobillacke handelt.

16. Formulierungen nach Anspruch 14, **dadurch gekennzeichnet, dass** es sich um kosmetische Formulierungen handelt.

17. Formulierungen nach einem oder mehreren der Ansprüche 14 bis 16, **dadurch gekennzeichnet, dass** sie neben dem beschichteten BiOCl-Pigment nach einem oder mehreren der Ansprüche 1 bis 7 mindestens einen Bestandteil ausgewählt aus der Gruppe Absorptionsmittel, Adstringenzien, antimikrobiellen Stoffe, Antioxidantien, Antiperspirantien, Antischaummittel, Antischuppenwirkstoffe, Antistatika, Bindemittel, biologischen Zusatzstoffe, Bleichmittel, Chelatbildner, Desodorierungsmittel, Emollentien, Emulgatoren, Emulsions-stabilisatoren, Farbstoffe, Feuchthaltemittel, Filmbildner, Füllstoffe, Geruchsstoffe, Geschmacksstoffe, Insect Repellents, Konservierungsmittel, Korrosionsschutzmittel, kosmetischen Öle, Lösungsmittel, Oxidationsmittel, pflanzlichen Bestandteile, Puffersubstanzen, Reduktionsmittel, Tenside, Treibgase, Trübungsmittel, UV-Filter, UV-Absorber, Vergällungsmittel, Aloe Vera, Avocadoöl, Coenzym Q10, grüner Tee Extrakt, Viskositätsregler, Parfüme, Vitamine, Enzyme, Spurenelemente, Proteine, Kohlenhydrate, organische Pigmente, anorganische Pigmente enthalten.

Europäisches
Patentamt

European
Patent Office

Office européen
des brevets

**EUROPÄISCHER RECHERCHENBERICHT**

Nummer der Anmeldung

EP 20 21 3109

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2011/105676 A1 (GAO YU [CN] ET AL) 5. Mai 2011 (2011-05-05) * Zusammenfassung * * Ansprüche 1,7 * * Beispiele 1-3 * * Absätze [0032], [0035], [0039] * ----- | 1-7, 12-17 | INV. C09C1/00 C08K9/04 A61K8/19 C08K9/02 C09D11/00 D21H17/67 |
| X | US 2017/321058 A1 (RUEGER REINHOLD [DE] ET AL) 9. November 2017 (2017-11-09) * Zusammenfassung * * Ansprüche 1,5 * * Absätze [0044], [0045], [0099], [0073] * ----- | 8-11 | |
| A | GAO FEIDAN ET AL: "Chemically bonded graphene/BiOCl nanocomposites as high-performance photocatalysts", PHYSICAL CHEMISTRY CHEMICAL PHYSICS, Bd. 14, Nr. 30, 1. Januar 2012 (2012-01-01), Seiten 10572-10578, XP055798628, ISSN: 1463-9076, DOI: 10.1039/c2cp41045a Gefunden im Internet: URL:https://pubs.rsc.org/en/content/articl epdf/2012/cp/c2cp41045a> [gefunden am 2021-04-23] * das ganze Dokument * ----- -/-- | 1-11 | RECHERCHIERTE SACHGEBIETE (IPC) C09C C08K D21H C09D A61Q A61K |

Der vorliegende Recherchenbericht wurde für alle Patentansprüche erstellt

| Recherchenort | Abschlußdatum der Recherche | Prüfer |
|---|---|---|
| Den Haag | 23. April 2021 | Mertins, Frédéric |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes
Dokument

EPO FORM 1503 03.82 (P04C03)

Europäisches
Patentamt

European
Patent Office

Office européen
des brevets

**EUROPÄISCHER RECHERCHENBERICHT**

Nummer der Anmeldung

EP 20 21 3109

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | ZHU LUPING ET AL: "In situ synthesis of N-doped carbon nanotubes-BiOCl nanocomposites and their synergistic photocatalytic performance", RSC ADVANCES, Bd. 6, Nr. 4, 1. Januar 2016 (2016-01-01), Seiten 2926-2934, XP055798629, DOI: 10.1039/C5RA24149A Gefunden im Internet: URL:https://pubs.rsc.org/en/content/articl epdf/2016/ra/c5ra24149a> [gefunden am 2021-04-23] * das ganze Dokument * ----- | 1-11 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| Den Haag | 23. April 2021 | Mertins, Frédéric |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes
    Dokument

EPO FORM 1503 03.82 (P04C03)

Seite 2 von 2

**ANHANG ZUM EUROPÄISCHEN RECHERCHENBERICHT
ÜBER DIE EUROPÄISCHE PATENTANMELDUNG NR.**

EP 20 21 3109

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-04-2021

| Im Recherchenbericht angeführtes Patentdokument | Datum der Veröffentlichung | Mitglied(er) der Patentfamilie | Datum der Veröffentlichung |
|---|---|---|---|
| US 2011105676 A1 | 05-05-2011 | CN 101784616 A | 21-07-2010 |
| | | EP 2178986 A1 | 28-04-2010 |
| | | JP 2010536968 A | 02-12-2010 |
| | | US 2011105676 A1 | 05-05-2011 |
| | | WO 2009024471 A1 | 26-02-2009 |
| US 2017321058 A1 | 09-11-2017 | CN 107124885 A | 01-09-2017 |
| | | DE 102014018276 A1 | 16-06-2016 |
| | | EP 3230385 A1 | 18-10-2017 |
| | | JP 6697465 B2 | 20-05-2020 |
| | | JP 2018506599 A | 08-03-2018 |
| | | KR 20170094330 A | 17-08-2017 |
| | | US 2017321058 A1 | 09-11-2017 |
| | | WO 2016091354 A1 | 16-06-2016 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0671161 A

- DE OS4308282 A